# EUROPÄISCHE PATENTANMELDUNG

(11) **EP 1 132 077 A2**
(43) Veröffentlichungstag der Anmeldung: **12.09.2001**
(21) Anmeldenummer: 01105143.0
(22) Anmeldetag: 02.03.2001
(51) Int. Cl.: A61K 7/08

(54) **Haarbehandlungsmittel**

(30) Priorität: 11.03.2000 DE 10012011
(71) Anmelder: Clariant GmbH, 65929 Frankfurt am Main (DE)
(72) Erfinder: Klein, Sonja, 65795 Hattersheim (DE)
(74) Vertreter: Otto, Adalbert, Dr.

(57) **Zusammenfassung**

Gegenstand der Erfindung sind Haarbehandlungsmittel, die mindestens eine quartäre Ammoniumverbindung und ein Perlglanzkonzentrat, enthaltend eine perlglanzgebende Komponente und nichtionische, amphotere und/oder zwitterionische Tenside und Wasser enthalten. In einer bevorzugten Ausführungsform enthalten die Perlglanzkonzentrate Titandioxid.

## Beschreibung

Die Erfindung betrifft Haarbehandlungsmittel, insbesondere Haarkuren und Haarspülungen, enthaltend quaternäre Ammoniumverbindungen, mit ausgezeichnetem Perlglanzeffekt.

Für die Reinigung der Haut und der Haare werden oberflächenaktive Stoffe eingesetzt, die insbesondere bei täglicher Anwendung den Nachteil haben, dass sie eine stark entfettende Wirkung haben, Haut und Haare schädigen und die Kämmbarkeit der Haare beeinträchtigen.
Bekannt ist, dass kationische Tenside, insbesondere quartäre Ammoniumverbindungen auf das durch Waschen, Dauerwellen und Färben geschädigte Haar aufziehen, dieses glätten und der statischen Aufladbarkeit beim Kämmen entgegenwirken.

Von Nachteil ist, dass Formulierungen aus quaternären Ammoniumverbindungen hinsichtlich ihres Erscheinungsbildes nicht brillant sind, sondern stumpf wirken und ästhetisch wenig ansprechend sind.

In US 5 019 376 werden kationische Öl-in-Wasser Emulsionen zur Pflege der Haare beansprucht, die bei Zugabe von (C₁₂-C₁₆)-Fettsäuren, (C₁₂-C₁₈)-Alkoholen und einem Verdicker, insbesondere Hydroxyethylcellulose, Perlglanzeffekte zeigen. Unbefriedigend ist eine nur mäßige Lagerstabilität der Mittel. Perlglanzkonzentrate geben reproduzierbaren Glanz und sind somit anwendungsfreundlicher.

EP 0 376 083 beschreibt, dass wässrigen Zubereitungen durch Zugabe eines Perlglanzkonzentrates aus perlglanzbildenden Komponenten, nichtionogenen Lösungsvermittlern, amphoteren und/oder zwitterionischen Tensiden und niedermolekularen, mehrwertigen Alkoholen, ein perlmuttartiger, seidiger Glanz verliehen werden kann.

Bisher gibt es nur wässrige, anionische Formulierungen, wie z.B. Shampoos, Duschbäder und Schaumbäder, auf dem Markt, die einen Perlglanzeffekt zeigen.
Es bestand daher der Wunsch nach kationisch aufgebauten Haarbehandlungsmitteln, insbesondere Haarkuren und Haarspülungen, mit einem brillanten Perlglanz.

Überraschenderweise wurde nun gefunden, dass Haarbehandlungsmittel, enthaltend mindestens eine quartäre Ammoniumverbindung und ein Perlglanzkonzentrat, enthaltend eine perlglanzgebende Komponente und nichtionische, amphotere und/oder zwitterionische Tenside und Wasser, einen hervorragenden Perlglanzeffekt zeigen.

Gegenstand der Erfindung sind somit Haarbehandlungsmittel, enthaltend mindestens eine quartäre Ammoniumverbindung und ein Perlglanzkonzentrat, enthaltend eine perlglanzgebende Komponente und nichtionische, amphotere und/oder zwitterionische Tenside und Wasser.

Als Ammoniumverbindung kommen bevorzugt solche Verbindungen in Frage, die eine oder zwei (C₈-C₂₀)-Alkyl- oder (C₈-C₂₀)-Alkenylgruppen enthalten. Die übrigen Gruppen sind (C₁-C₄)-Alkyl- oder (C₂-C₄)-Hydroxyalkylgruppen. Besonders bevorzugte Verbindungen sind Cetyltrimethylammoniumchlorid, PEG-5-Stearyllactat, Stearyltrimethylammoniumchlorid, Behenyltrimethylammoniumchlorid, Polydiallyldimethylammoniumchlorid, Distearyldimethylammoniumchlorid, Distearoylethyldimethylammoniumchlorid, Distearoylethylhydroxyethylammonium-Methosulfat und/oder Dicocoylethylhydroxyethylammonium-Methosulfat.

Die erfindungsgemäßen Haarbehandlungsmittel enthalten bevorzugt 0,1 bis 15, besonders bevorzugt 1 bis 10 Gew.-%, an quartären Ammoniumverbindungen.

Die erfindungsgemäßen Haarbehandlungsmittel enthalten bevorzugt 0,5 bis 20, besonders bevorzugt 2 bis 7 und insbesondere bevorzugt 3 bis 6 Gew.-% an Perlglanzkonzentrat.

Die Perlglanzkonzentrate bestehen hauptsächlich aus einer perlglanzgebenden Komponente, einem oder mehreren Tensiden und Wasser.

Die Perlglanzkonzentrate enthalten bevorzugt 5 bis 40, besonders bevorzugt 20 bis 30 Gew.-% der perlglanzgebenden Komponente und bevorzugt 5 bis 55 und besonders bevorzugt 15 bis 30 Gew.-% an nichtionischen, amphoteren und/oder zwitterionischen Tensiden.

In einer bevorzugten Ausführungsform enthalten die Perlglanzkonzentrate zusätzlich noch Titandioxid, das den Perlglanzeffekt erhöht.

Bevorzugt enthalten die Perlglanzkonzentrate 0,01 bis 10 Gew.-%, besonders bevorzugt 0,05 bis 5 Gew.-%, insbesondere bevorzugt 0,1 bis 2 Gew.-%, an Titandioxid.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z.B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykoldistearate mit durchschnittlich 3 Glykoleinheiten.

Die erfindungsgemäß eingesetzten Perlglanzkonzentrate können neben der perlglanzgebenden Komponente und den Tensiden zusätzlich noch bis zu 10 Gew.-% an mehrwertigen (C₂-C₈)-Alkoholen enthalten. Als mehrwertige Alkohole sind (C₂-C₆)-Polyole bevorzugt, insbesondere Ethylenglykol, 1,2- und 1,3-Propylenglykol, Glycerin, Di- und Triethylenglykol, Erythrit, Arabit, Adonit, Xylit, Sorbit, Mannit und Dulcit.

Erfindungsgemäß werden die Perlglanzmittel als Perlglanzkonzentrate den Formulierungen zugemischt. Die Herstellung derartiger Perlglanzkonzentrate ist in EP 0 376 083 beschrieben.

Als Tenside können nichtionogene, amphotere und/oder zwitterionische grenzflächenaktive Verbindungen verwendet werden, die sich durch eine lipophile, bevorzugt lineare Alkyl- oder Alkenylgruppe und mindestens eine hydrophile Gruppe auszeichnen. Diese hydrophile Gruppe kann sowohl eine ionogene als auch eine nichtionogene Gruppe sein.

Nichtionogene Tenside enthalten als hydrophile Gruppe z.B. eine Polyolgruppe, eine Polyolalkenylethergruppe oder eine Kombination aus Polyol- und Polyglykolethergruppen.
Bevorzugt sind Anlagerungsprodukte von 2 bis 30 Mol Ethylenoxid, Anlagerungsprodukte von 2 bis 30 Mol Ethylenoxid und bis 5 Mol Propylenoxid oder Anlagerungsprodukte von bis 5 Mol Propylenoxid an lineare Fettalkohole mit 8 bis 22 C-Atomen und an Alkylphenole mit 8 bis 15 C-Atomen in der Alkylgruppe, (C₁₂-C₁₉)-Fettsäuremono- und diester von Anlagerungsprodukten von 1 bis 30 Mol Ethylenoxid an Glycerin, Glycerinmono- und -diester und Sorbitanmono- und-diester von gesättigten und ungesättigten (C₈-C₁₈)-Fettsäuren und deren Ethylenoxidanlagerungsprodukte, (C₈-C₁₈)-Alkylmono- und ―oligoglykoside und deren ethoxylierte Analoga, Anlagerungsprodukte von 10 bis 60 Mol Ethylenoxid an Ricinusöl und gehärtetes Ricinusöl, ethoxylierte und nicht ethoxylierte Mono-, Di-und Trialkylmonophosphor-säureester, insbesondere Mono-, Di- und Tri-(Lauryltetraglykolether)-o-Phosphor-säureester und Mono-, Di- und Tri-(Cetyltetraglykolether)-o-Phosphorsäureester.

Unter amphoteren Tensiden werden solche oberflächenaktive Verbindungen verstanden, die außer einer (C₈-C₁₈)-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete amphotere Tenside sind N-Acylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe. Besonders bevorzugte amphotere Tenside sind das N-Kokosalkylaminopropionat, das Kokosacylaminoethylaminopropionat und (C₁₂-C₁₈)-Alkylsarcosine.
Unter zwitterionischen Tensiden werden solche oberflächenaktive Verbindungen verstanden, die im Molekül mindestens eine quartäre Ammoniumgruppe und mindestens eine -COO- oder eine SO₃-Gruppe tragen. Besonders geeignete zwitterionische Tenside sind Betaine, wie beispielsweise die N-Alkyl-N,N-dimethylammoniumglycinate, z.B. Kokosalkyldimethylammoniumglycinate, N-Acylaminopropyl-N,N-dimethylammoniumglycinate, z.B. Kokosacylaminopropyldimethylammoniumglycinat, 2-Alkl-3-carboxymethyl-3-hydroxyethylimidazoline mit jeweils 8 bis 18 C-Atomen in der Alkyl- oder Acylgruppe und das Kokosacylaminoethylhydroxyethylcarboxymethylglycinat.

Die erfindungsgemäßen Mittel können mehrere der genannten Tenside enthalten. Bevorzugte Tensid-Mischungen sind Mischungen aus nichtionogenen und zwitterionischen oder amphoteren Tensiden in einem Gewichtsverhältnis von 5 zu 1 bis 1 zu 5 oder Mischungen aus nichtionogenen Tensiden und beliebigen Mischungen aus zwitterionischen und amphoteren Tensiden in einem Gewichtsverhältnis von 5 zu 1 bis 1 zu 5.

Die erfindungsgemäßen Mittel können weitere Zusatz- und Hilfsstoffe, wie Ölkörper, Überfettungsmittel, Stabilisatoren, Konsistenzgeber, weitere Verdickungsmittel, Kationpolymere, Siliconverbindungen, biogene Wirkstoffe, Antischuppenmittel, Filmbildner, Konservierungsmittel, Hydrotrope, feuchtigkeitsspendende Substanzen, Solubilisatoren, UV-Absorber, Farb- und Duftstoffe enthalten.

Als Ölkörper eignen sich alle bekannten Öle, Fette und Wachse mineralischer, tierischer, pflanzlicher und synthetischer Herkunft. Bevorzugt sind als Öl- und Fettkomponenten Diallylether mit insgesamt 12 bis 24 C-Atomen, Fettsäureester mit insgesamt 12 bis 26 C-Atomen, flüssige Kohlenwasserstoffe mit 10 bis 32 C-Atomen und Gemische davon. Geeignete Fettsäureester sind z.B. Methylpalmitat, Ethyloleat, Isopropylmyristat, n-l lexyllaurat, n-Butylstearat und Cetyl-/ Stearylisononanoat. Besonders bevorzugt sind Paraffinöle, Vaseline, Pflanzenöle, synthetische Triglyceride wie z.B. Glyceryltricaprylat, sowie Silikonöle.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden. Als Konsistenzgeber kommen Fettalkohole mit 12 bis 22, vorzugsweise 12 bis 18 Kohlenstoffatomen sowie Partialglyceride in Betracht.

Als weitere Verdickungsmittel können Polysaccharide, insbesondere Xantham-Gum, Guar-Guar, Agar-Agar, Alginate, Carboxymethylcellulose, Hydroxyethylcellulose, höhermolekulare Polyethylenglykolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate oder Alkyloligoglucoside, sowie Elektrolyte wie Kochsalz und Ammoniumchlorid eingesetzt werden.

Geeignete Siliconverbindungen sind beispielsweise Dimethylpolysiloxan, Methylphenylpolysiloxane, cyclische Silicone sowie amino-, fettsäure-, alkohol-, polyether-, epoxy-, fluor- und/ oder alkylmodifizierte Siliconverbindungen, die bei Raumtemperatur sowohl flüssig als auch harzförmig vorliegen können.

Unter biogenen Wirkstoffen sind beispielsweise Bisabolol® , Allantoin® , Phytantriol® , Panthenol® , AHA-Säuren, Pflanzenextrakte und Vitaminkomplexe zu verstehen.

Als Antischuppenmittel können Climbazol® , Octopirox® , Oxiconazol® und Zinkpyrethion® eingesetzt werden.

Gebräuchliche Filmbildner sind Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Zur Verbesserung des Fließverhaltens können ferner Hydrotrope wie beispielsweise Ethanol, Isopropylalkohol, Propylenglykol oder Glucose eingesetzt werden.

Als Konservierungsmittel eignen sich beispielsweise Phenoxyethanol, Formaldehydlösung, Parabene, Pentandiol oder Sorbinsäure.

Als feuchtigkeitsspendende Substanz stehen beispielsweise Isopropylpalmitat, Glycerin und/oder Sorbitol zu Verfügung.

Der Gesamtanteil an Hilfs- und Zusatzstoffen in den Mitteln beträgt bevorzugt 1 bis 50 und besonders bevorzugt 5 bis 40 Gew.-%.

Die Brillanz und Stabilität des Perlglanzes der erfindungsgemäßen Mittel ist unabhängig vom pH-Wert und der Viskosität, sowie von den Einsatzkonzentrationen der Einzelkomponenten, sofern diese innerhalb der oben genannten Einsatzmengen liegen.

Bevorzugt handelt es sich bei den Haarbehandlungsmitteln um Haarspülungen oder Haarkuren.

### Beispiele

Im folgenden werden eine Reihe von erfindungsgemäßen Formulierungen angegeben. Alle Prozentangaben sind Gewichtsprozente. Die Beispiele dienen der näheren Erläuterung der Erfindung, ohne sie jedoch einzuschränken.

Neben den in den jeweiligen Beispielen angegebenen Komponenten enthalten die Formulierungen noch Wasser sowie geringe Mengen Parfümöl, Konservierungsmittel, und Farbstofflösung ad 100 Gew.-% der Formulierung.

| Beispiel 1: Cremespülung | |
|---|---|
| Genamin KSL® | 7 % |
| Hostaphat KL 340 D® | 1,5 % |
| Genapol PDB® | 4 % |
| Tylose H 100000YP2® | 1,5 % |
| Paraffinöl nv | 1 % |

| Beispiel 2: Haarkur | |
|---|---|
| Genamin KSL® | 7 % |
| Hostaphat KL 340 D® | 1,5 % |
| Genapol PDB® | 4 % |
| Tylose H 100000YP2® | 1,7 % |
| Paraffinöl nv | 1 % |
| Jojobaöl | 1% |
| Propylenglykol | 0,8 % |
| Isopropylpalmitat | 1 % |
| Dow Corning 190® | 0,8 % |
| Extrapon® | 0,3 % |
| Vitamin E | 0,3 % |
| Panthenol (Vitamin B 5) | 0,5 % |

| Beispiel 3: Cremespülung | |
|---|---|
| Genamin STAC® | 3,5 % |
| Hostacerin T-3® | 1,5 % |
| Genapol PDB® | 4 % |
| Tylose H 100000YP2® | 1,5% |
| Paraffinöl nv | 1 % |

| Beispiel 4: Cremespülung | |
|---|---|
| Genamin CTAC® | 6 % |
| Hostacerin T- 3® | 1,5 % |
| Genapol PDB® | 3 % |
| Tylose H 100000YP2® | 1,2 % |

| Beispiel 5: Cremespülung | |
|---|---|
| Genamin KSL® | 7 % |
| Hostaphat KL 340 D® | 1,5 % |
| Euperlan PK 3000® | 4 % |
| Tylose H 100000YP2® | 1,5% |
| Paraffinöl nv | 1% |

| Beispiel 6: Haarkur | |
|---|---|
| Genamin KSL® | 9 % |
| Genamin KDM- P® | 1,5 % |
| Hostaphat KL 340 D® | 1,5 % |
| Genapol PDB® | 4 % |
| Tylose H 100000YP2® | 1,5 % |
| Jojobaöl | 1 % |
| Panthenol (Vitamin B 5) | 0,5 % |

### Herstellung der Formulierungen:

Die Herstellung erfolgt in den aufeinanderfolgenden Schritten I bis VI:
I Tylose im Wasser bei RT unter Rühren aufquellen
II Ölphase, enthaltend Öl/e, Quats, Lösungsvermittler und gegebenenfalls Vitamine bei ca. 75°C aufschmelzen
III Wasserphase (I) auf ca. 75°C erhitzen
IV Wasserphase (I) zur Ölphase (II) geben und kaltrühren
V Bei ca. 30°C Perlglanzkonzentrat, evt. Farbstoff, Parfüm und Pflanzenextrakte zugeben
VI pH-Wert einstellen

| INCI Bezeichnung der eingesetzten Handelsprodukte: | | |
|---|---|---|
| Genamin KDM-P® | (Clariant) | Behenyltrimethylammonium Chlorid |
| Genamin KSL® | (Clariant) | PEG- 5 Stearyl Ammonium Lactat |
| Genamin CTAC® | (Clariant) | Cetyltrimethylammonium Chlorid |
| Genamin STAC® | (Clariant) | Stearyltrimethylammonium Chlorid |
| Tylose H 100000 YP2® | (Clariant) | Hydroxyethylcellulose |
| Genapol PDB® | (Clariant) | Glycol Distearat, Laureth- 4, Cocamidopropyl Betain |
| Hostaphat KL 340 D® | (Clariant) | Trilaureth-4 Phosphat |
| Hostacerin T- 3® | (Clariant) | Ceteareth-3 |
| Dow Corning 190® | (Dow corning) | Dimethicon Copolyol |
| Euperlan PK 3000® | (Henkel) | Glycol Distearat, Laureth-4, Cocamidopropyl Betain |
| Extrapon® | (Dragoco) | Pflanzliche Extrakte |

## Patentansprüche

1. Haarbehandlungsmittel, enthaltend eine oder mehrere quartäre Ammoniumverbindungen und ein oder mehrere Perlglanzkonzentrate, enthaltend eine oder mehrere perlglanzgebende Komponenten und nichtionische, amphotere und/oder zwitterionische Tenside und Wasser.

2. Haarbehandlungsmittel nach Anspruch 1, **dadurch gekennzeichnet, dass** sie 0,1 bis 15 Gew.-% an quartären Ammoniumverbindungen enthalten.

3. Haarbehandlungsmittel nach mindestens einem der Ansprüche 1 bis 2, **dadurch gekennzeichnet, dass** sie 0,5 bis 20 Gew.-% an Perlglanzkonzentrat enthalten.

4. Haarbehandlungsmittel nach mindestens einem der Ansprüche 1 bis 3, **dadurch gekennzeichnet, dass** die Perlglanzkonzentrate 5 bis 40 Gew.-% der perlglanzgebenden Komponente und 5 bis 55 Gew.-% an nichtionischen, amphoteren und/oder zwitterionischen Tensiden enthalten.

5. Haarbehandlungsmittel nach mindestens einem der Ansprüche 1 bis 4, **dadurch gekennzeichnet, dass** die Perlglanzkonzentrate Titandioxid enthalten.

6. Haarbehandlungsmittel nach Anspruch 5, **dadurch gekennzeichnet, dass** die Perlglanzkonzentrate 0,01 bis 10 Gew.-% Titandioxid enthalten.

7. Haarbehandlungsmittel nach mindestens einem der Ansprüche 1 bis 6, **dadurch gekennzeichnet, dass** sie als quartäre Ammoniumverbindungen Cetyltrimethylammoniumchlorid, PEG-5-Stearyllactat, Stearyltrimethylammoniumchlorid, Behenyltrimethylammoniumchlorid, Polydiallyldimethylammoniumchlorid, Distearyldimethylammoniumchlorid, Distearoylethyldimethylammoniumchlorid, Distearoylethylhydroxyethylammoniummethosulfat und/oder Dicocoyl-ethylhydroxyethylammonium-methosulfat enthalten.

8. Haarbehandlungsmittel nach mindestens einem der Ansprüche 1 bis 7, **dadurch gekennzeichnet, dass** sie als perlglanzgebende Komponente Fettsäuremonoalkanolamide, Fettsäuredialkanolamide, Monoester oder Diester von Alkylenglykolen, Fettsäuren und deren Metallsalze, Monoester oder Polyester von Glycerin mit Carbonsäuren und/oder Ketosulfone enthalten.

9. Haarbehandlungsmittel nach Anspruch 8, **dadurch gekennzeichnet, dass** sie als perlglanzgebende Komponente Ethylenglykoldistearate und/oder Polyethylenglykoldistearate mit durchschnittlich 3 Glykoleinheiten enthalten.

10. Haarbehandlungsmittel nach mindestens einem der Ansprüche 1 bis 9, **dadurch gekennzeichnet, dass** sie als nichtionische Tenside Anlagerungsprodukte von 2 bis 30 Mol Ethylenoxid, Anlagerungsprodukte von 2 bis 30 Mol Ethylenoxid und bis 5 Mol Propylenoxid oder Anlagerungsprodukte von bis 5 Mol Propylenoxid an lineare Fettalkohole mit 8 bis 22 C-Atomen und an Alkylphenole mit 8 bis 15 C-Atomen in der Alkylgruppe, (C₁₂-C₁₉)-Fettsäuremono- und diester von Anlagerungsprodukten von 1 bis 30 Mol Ethylenoxid an Glycerin, Glycerinmono- und -diester und Sorbitanmono- und -diester von gesättigten und ungesättigten (C₈-C₁₈)-Fettsäuren und deren Ethylenoxidanlagerungsprodukte, (C₈-C₁₈)-Alkylmono- und -oligoglykoside und deren ethoxylierte Analoga, Anlagerungsprodukte von 10 bis 60 Mol Ethylenoxid an Ricinusöl und gehärtetes Ricinusöl und/oder ethoxylierte und nicht ethoxylierte Mono-, Di- und Trialkylmonophosphorsäureester, als amphotere Tenside N-Acylglycine, N-Alkylpropionsäuren, N-Alkylaminobuttersäuren, N-Alkyliminodi-propionsäuren, N-Hydroxyethyl-N-alkylamidopropylglycine, N-Alkyltaurine, N-Alkylsarcosine, 2-Alkylaminopropionsäuren und/oder Alkylaminoessigsäuren mit jeweils 8 bis 18 C-Atomen in der Alkylgruppe und als zwitterionische Tenside N-Alkyl-N,N-dimethylammoniumglycinate, N-Acyl-aminopropyl-N,N-dimethyl-ammoniumglycinate, 2-Alkyl-3-carboxylmethyl-3-hydroxyethylimidazoline mit jeweils 8 bis 18 C-Atomen in der Alkyl- oder Acylgruppe und/oder Kokosacylaminoethylhydroxyethylcarboxymethylglycinat enthalten.

11. Haarbehandlungsmittel nach mindestens einem der Ansprüche 1 bis 10, **dadurch gekennzeichnet, dass** sie zusätzlich noch Ölkörper, Überfettungsmittel, Stabilisatoren, Konsistenzgeber, weitere Verdickungsmittel, Kationpolymere, Siliconverbindungen, biogene Wirkstoffe, Antischuppenmittel, Filmbildner, Konservierungsmittel, Hydrotrope, feuchtigkeitsspendende Substanzen, Solubilisatoren, UV-Absorber, Farbstoffe und/oder Duftstoffe enthalten.

12. Haarbehandlungsmittel nach mindestens einem der Ansprüche 1 bis 11, **dadurch gekennzeichnet, dass** sie als Verdicker Hydroxyethylcellulose enthalten.

13. Haarbehandlungsmittel nach mindestens einem der Ansprüche 1 bis 12, **dadurch gekennzeichnet, dass** es sich dabei um eine Haarkur oder eine Haarspülung handelt.
